# EUROPÄISCHE PATENTSCHRIFT

(11) **EP 0 988 293 B1**
(45) Veröffentlichungstag und Bekanntmachung des Hinweises auf die Patenterteilung: **17.04.2002**
(21) Anmeldenummer: 98930765.7
(22) Anmeldetag: 29.05.1998
(51) Int. Cl.: C07D 249/12, C07D 261/12, A01N 43/653, A01N 43/80

(54) **BISIMINOSUBSTITUIERTE PHENYLVERBINDUNGEN UND IHRE VERWENDUNG ALS SCHÄDLINGSBEKÄMPFUNGSMITTEL**
BISIMINO-SUBSTITUTED PHENYL COMPOUNDS AND THEIR USE AS PESTICIDES
COMPOSES PHENYLE SUBSTITUES PAR BISIMINO ET LEUR UTILISATION COMME AGENTS DE LUTTE CONTRE LES PARASITES

(30) Priorität: 09.06.1997 DE 19724200
(43) Veröffentlichungstag der Anmeldung: 29.03.2000
(73) Patentinhaber: BASF AKTIENGESELLSCHAFT, 67056 Ludwigshafen (DE)
(72) Erfinder: GRAMMENOS, Wassilios, D-67063 Ludwigshafen (DE); BAYER, Herbert, D-68159 Mannheim (DE); GROTE, Thomas, D-67105 Schifferstadt (DE); SAUTER, Hubert, D-68167 Mannheim (DE); GYPSER, Andreas, D-68159 Mannheim (DE); KIRSTGEN, Reinhard, D-67434 Neustadt (DE); MÜLLER, Bernd, D-67227 Frankenthal (DE); PTOCK, Arne, D-67067 Ludwigshafen (DE); RÖHL, Franz, D-67105 Schifferstadt (DE); LORENZ, Gisela, D-67434 Neustadt (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(74) Vertreter: Fitzner, Uwe, Dr.
(86) Internationale Anmeldenummer: EP9803229
(87) Internationale Veröffentlichungsnummer: WO9856774

(56) Entgegenhaltungen:
- WO-A-97/02255
- DE-A- 19 539 324

## Beschreibung

Die vorliegende Erfindung betrifft bisiminosubstituierte Phenylverbindungen der Formel I, 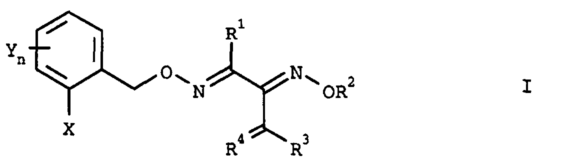 in der die Substituenten folgende Bedeutung haben:
- X: eine Gruppe A oder B, 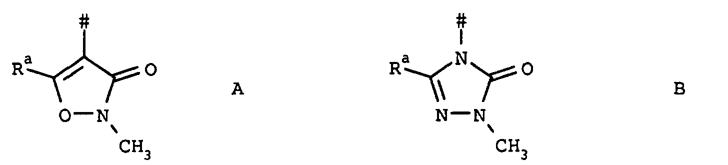 wobei
# die Bindung mit dem Phenylring kennzeichnet und
R^{a} für Halogen, C₁-C₄-Alkyl oder C₁-C₄-Alkoxy steht;
- Y: Halogen, C₁-C₄-Alkyl, C₁-C₄-Halogenalkyl oder C₁-C₄-Alkoxy;
- n: 0, 1 oder 2, wobei die Reste Y verschieden sein können, wenn n = 2 ist;
- R¹: C₁-C₄-Alkyl;
- R²: C₁-C₄-Alkyl, C₁-C₄-Halogenalkyl, C₃-C₄-Alkenyl, C₃-C₄-Halogen-alkenyl, C₃-C₄-Alkinyl oder C₃-C₄-Halogenalkinyl;
- R³: Wasserstoff, C₁-C₄-Alkyl, C₁-C₄-Halogenalkyl, Phenyl oder partiell oder vollständig halogeniertes Phenyl oder Phenyl mit 1-4 der folgenden Reste: Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio;
- R⁴: =CR^{b}R^{c} oder =N-OR^{d}, wobei
- R^{b}, R^{c}: unabhängig voneinander Wasserstoff, C₁-C₆-Alkyl, Phenyl oder partiell oder vollständig halogeniertes Phenyl oder Phenyl mit 1-4 der folgenden Reste: Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio bedeuten; und
- R^{d}: einer der bei R² genannten Reste ist.

Zusätzlich betrifft die Erfindung Verfahren und Zwischenprodukte zur Herstellung der Verbindungen I, sowie Mittel und die Verwendung der Verbindungen I zur Bekämpfung von Schadpilzen und tierischen Schädlingen.

4-Phenyl-2,3-dihydro-isoxazolone und 4-Phenyl-2,4-dihydrotriazolone mit orthoständiger Methoximino-Gruppe werden in WO-A 97/02,255 offenbart.

α-Phenylacrylsäure- und α-Phenyl-α-methoximinoessigsäurederivate mit orthoständiger Bisoximether-Gruppierung sind in WO-A 95/21,153 und WO-A 97/05,103, und ebensolche mit Trisoximether-Gruppierung in DE 19539324 beschrieben.

Die in den vorstehend genannten Schriften beschriebenen Verbindungen sind als Pflanzenschutzmittel gegen Schadpilze und z.T. gegen tierische Schädlinge geeignet.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die Phenylverbindungen der Formel I gefunden. Weiterhin wurden Zwischenprodukte und Verfahren zur Herstellung der Verbindungen I, sowie die Verwendung der Verbindungen I und diese enthaltene Mittel zur Bekämpfung von Schadpilzen und tierischen Schädlingen gefunden. Die fungizide Wirkung ist bevorzugt.

Die Verbindungen der Formel I unterscheiden sich von den aus der oben genannten Schrift WO-A 97/02,255 bekannten Verbindungen in der Substitution der Oximinogruppe durch den an eine Doppelbindung gebundenen Rest R⁴, der nicht Wasserstoff sein kann. Die Verbindungen der Formel I weisen eine gegenüber den bekannten Verbindungen erhöhte Wirksamkeit gegen Schadpilze und tierische Schädlinge auf.

Die Verbindungen der Formel I können an sich analog der in WO-A 97/02,255, bzw. WO-A 95/21,153 und WO-A 97/05,103, sowie in DE 19539324 beschriebenen Methoden erhalten werden.

Die Verbindungen I können auf verschiedenen Wegen erhalten werden, wobei es für die Synthese unerheblich ist, ob zunächst die Gruppe X oder die Oximether-Gruppierung aufgebaut wird. Zur besseren Übersichtlichkeit wird in den nachfolgenden Reaktionsbeschreibungen daher die Bezeichnung X^{#} für den Rest X, bzw. eine geeignete Vorstufe dieses Restes und E^{#} für die Oximether-Gruppierung, bzw. eine geeignete Vorstufe dafür verwendet.

Insbesondere erhält man Verbindungen der Formel I.1 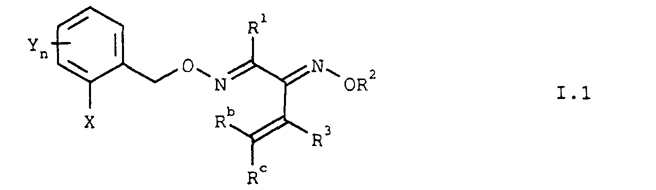 dadurch, daß man eine Benzylverbindung der Formel II^{#}, mit einem Oxim der Formel III umsetzt und den erhaltenen Oximether der Formel IV^{#} durch Halogenierung in die Halogenverbindung der Formel V^{#} überführt, V^{#} mit einem Hydroxylaminether der Formel VI' zum Bisoxim der Formel VI^{#} umsetzt, VI^{#} zur Carbonylverbindung der Formel VII^{#} oxidiert und VII^{#} mit einem Phosphor-Reagenz im Sinne einer Wittig-Reaktion zu einer Verbindung der Formel I.1^{#} umgesetzt wird.

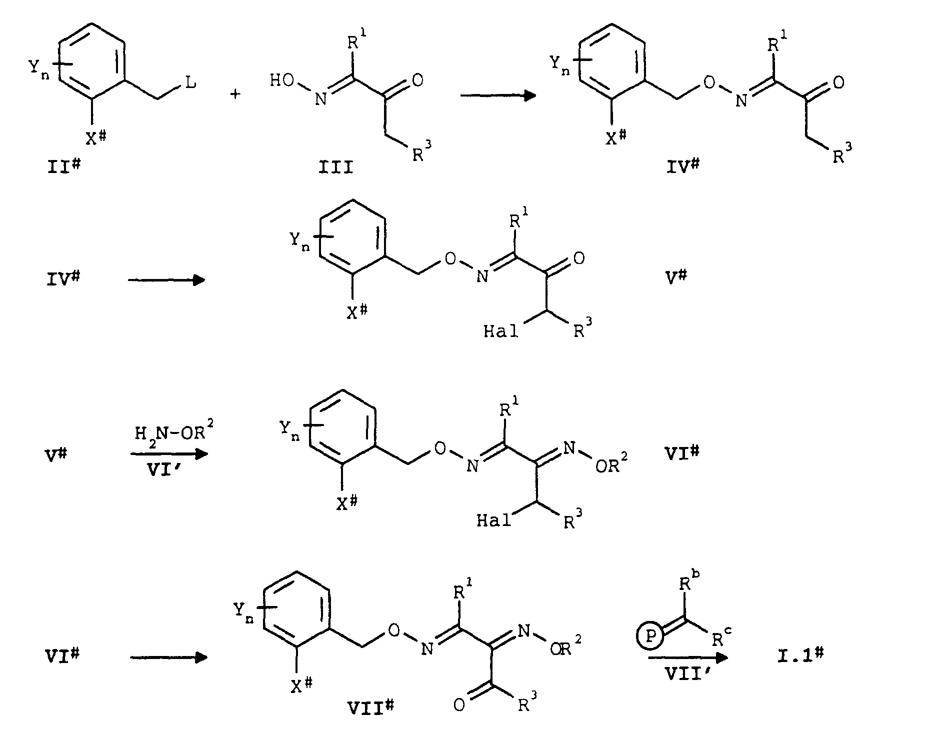

In Formel II^{#} steht L für eine nucleophil austauschbare Gruppe, beispielsweise Halogen, wie Fluor, Chlor, Brom oder Jod, insbesondere Chlor oder Brom, oder Alkyl- oder Arylsulfonate, wie Mesylat oder Tosylat.

In dem voranstehenden Reaktionsschema steht Ⓟ in der Formel VII' für einen Phosphoranylrest, wie beispielsweise Triphenylphosphoranyl.
1. Die Umsetzung der Benzylverbindung II^{#} mit dem Oxim der Formel III^{#} erfolgt in an sich bekannter Weise bei Temperaturen von -10°C bis 100°C, vorzugsweise 10°C bis 85°C, in einem inerten organischen Lösungsmittel in Gegenwart einer Base [vgl. WO-A 97/02,255; WO-A 96/36,229].
   Geeignete Lösungsmittel sind Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Tetrahydrofuran, Acetonitril und Dimethylformamid. Es können auch Gemische der genannten Lösungsmittel verwendet werden.
   Als Basen kommen allgemein anorganische Verbindungen, wie Alkalimetall- und Erdalkalimetallhydroxide wie Lithiumhydroxid, Natriumhydroxid, Kaliumhydroxid und Calziumhydroxid, Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat, sowie Alkalimetallhydrogencarbonate, wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine, wie Collidin, Lutidin und 4-Dimethylaminopyridin, sowie bicyclische Amine in Betracht. Besonders bevorzugt werden Natriumhydrid, Kaliumcarbonat und Natriummethanolat.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, III in einem Überschuß bezogen auf II^{#} einzusetzen.
   Die für die Herstellung der Verbindungen I benötigten Benzylverbindungen II^{#} sind aus der Literatur bekannt [vgl. WO-A 97/02,255]. Sie können auf dem folgenden Syntheseweg erhalten werden: 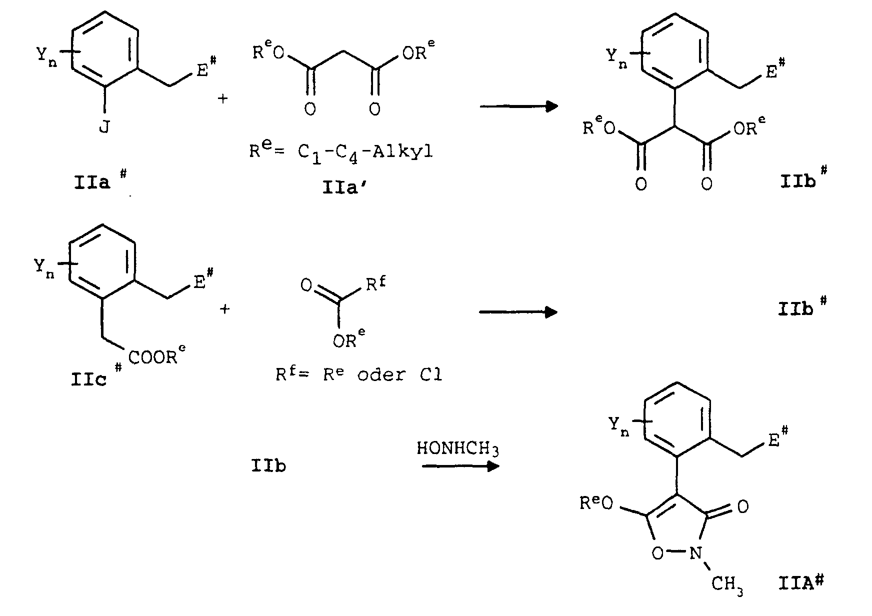 Verbindungen der Formel IIA^{#}, in denen R^{a} für Alkyl steht, erhält man auf dem aus US 4,952,573 bekannten Weg aus entsprechenden Phenylessigsäureestern IIC^{#}. 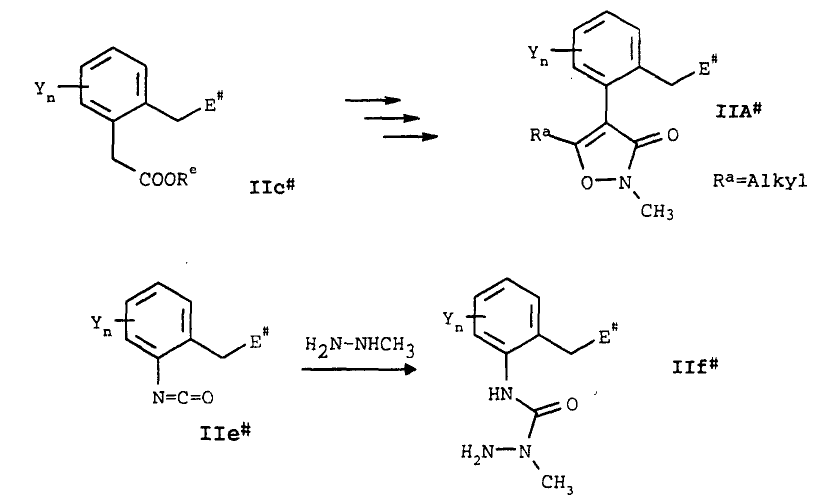 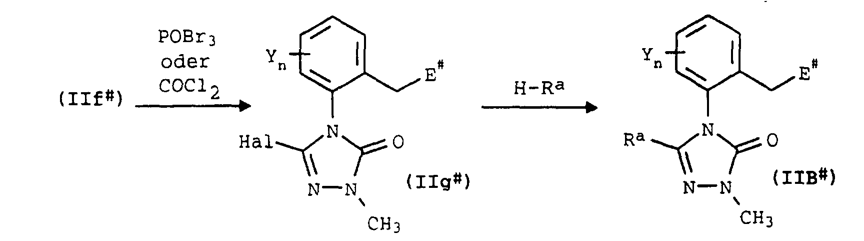
   Dieser Weg ist nicht nur zur Herstellung der Benzylverbindungen II^{#}, sondern prinzipiell auf jeder Synthesestufe der Oximether-Gruppierung E^{#} für den Aufbau der Gruppen A, bzw. B geeignet. Besonders bevorzugt erfolgt der Aufbau der Gruppierung X auf der Stufe der Verbindungen IIa oder IIe, in denen E^{#} für Wasserstoff steht.
   Die für die Herstellung der Verbindungen IB, in denen R^{a} für Alkyl steht, benötigten Benzylverbindungen IIB^{#} sind aus der Literatur bekannt [vgl. WO-A 96/36229], bzw. sind analog der Literatur [vgl. J. Org. Chem., Bd. 43 (1978) S. 936] zugänglich. Sie können durch Umsetzung der Carbamate der Formel IIf^{#} mit Orthoestern erhalten werden: 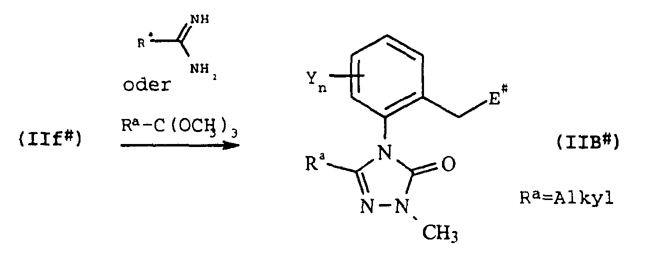 Die Oxime der Formel III sind ebenfalls aus der Literatur bekannt [vgl. WO-A- 95/21153] oder können gemäß der zitierten Literatur hergestellt werden.
2. Die Halogenierung des Oximethers IV^{#} erfolgt üblicherweise bei Temperaturen von -10°C bis 80°C, vorzugsweise 0°C bis 65°C, in einem inerten organischen Lösungsmittel gegebenenfalls in Gegenwart einer einer Säure [vgl. J. Org. Chem., S. 2532 (1981); Org. Synth., Bd. 55, S. 24 (1976); Tetrahedron S. 5611 (1970)].
   Als Halogenierungsmittel kommen Brom, Chlor, Pyridin*HBr₃, CuBr₂ und SO₂Cl₂ in Betracht, insbesondere Brom, CuBr₂ und SO₂Cl₂. Sie werden im allgemeinen in äquimolaren Mengen miteinander umgesetzt. Es kann für die Ausbeute vorteilhaft sein, sie in einem 1,2- bis 2,5-fachen Überschuß, bezogen auf die Verbindung IV^{#}, einzusetzen.
   Geeignete Lösungsmittel sind aromatische Kohlenwasserstoffe, wie Toluol, o-, m- und p-Xylol, halogenierte Kohlenwasserstoffe wie Methylenchlorid, Chloroform und Chlorbenzol, sowie Alkohole wie Methanol, Ethanol, n-Propanol, Isopropanol, n-Butanol und tert.-Butanol, besonders bevorzugt Cyclohexan, Methylenchlorid, Chloroform, Chlorbenzol und Methanol. Es können auch Gemische der genannten Lösungsmittel verwendet werden.
   Als Säuren und saure Katalysatoren finden anorganische Säuren wie Salzsäure, Bromwasserstoffsäure und Schwefelsäure, LewisSäuren wie Bortrifluorid, Aluminiumtrichlorid, Eisen-IIIchlorid, Zinn-IV-chlorid, Titan-IV-chlorid und Zink-IIchlorid, sowie organische Säuren wie Ameisensäure, Essigsäure, Propionsäure, Oxalsäure, Zitronensäure und Trifluoressigsäure Verwendung.
   Die Säuren werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, das Halogenierungsmittel in einem Überschuß bezogen auf IV^{#} einzusetzen.
3. Die Umsetzung des Oximethers V^{#} mit dem Hydroxylaminether der Formel VI' zu dem Bisoximether VI^{#} erfolgt in bekannter Weise bei Temperaturen von 0°C bis 85°C, vorzugsweise bei 20°C bis 65°C, in einem inerten organischen Lösungsmittel.
4. Die Oxidation des Bisoximethers VI# erfolgt in bekannter Weise bei Temperaturen von 20°C bis 160°C, vorzugsweise bei 20°C bis 100°C, in einem inerten organischen Lösungsmittel gegebenenfalls in Gegenwart einer Base [vgl. Houben-Weyl, Methoden der organischen Chemie, Band E3, S. 247 - 265, Georg Thieme Verlag, Stuttgart 1983].
   Als Oxidationsmittel kommen beispielsweise N-Methylmorpholin-N-Oxid, 2-Benzoyl-1-trifluormethansulfonyl-hydrazin, Trimethylamin-N-Oxid und Pyridin-N-Oxid in Frage, insbesondere N-Methylmorpholin-N-Oxid, Trimethylamin-N-Oxid und Pyridin-N-Oxid.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, außerdem organische Basen, z.B. tertiäre Amine wie Trimethylamin, Triethylamin, Triisopropylethylamin und N-Methylpiperidin, Pyridin, substituierte Pyridine wie Collidin, Lutidin und 4-Dimethylaminopyridin, sowie bicyclische Amine in Betracht. Besonders bevorzugt werden Natriumhydroxid, Natriumhydrogencarbonat und Kaliumcarbonat.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, das Oxidationsmittel in einem Überschuß bezogen auf VI^{#} einzusetzen.
5. Die Wittig-Reaktion erfolgt in bekannter Weise bei Temperaturen von -78°C bis 85°C, vorzugsweise -10°C bis 65°C, in einem inerten organischen Lösungsmittel in Gegenwart einer Base [vgl. EP-A 513 580].
   Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischenund Endprodukte als Feststoffe erhalten werden, kann die Reinigung auch durch Umkristallisieren oder Digerieren erfolgen.
   Die für die Herstellung der Verbindungen I benötigten Ausgangsstoffe der Formel III sind in der Literatur bekannt [J. Org. Chem., S. 2605 (1991); Bull. Soc. Chim. Fr., S. 1452 (1973)] oder können gemäß der zitierten Literatur hergestellt werden. Verbindungen der Formel I.2 erhält man beispielsweise dadurch, daß man eine Carbonylverbindung der Formel VI^{#} mit einem Hydroxylaminether der Formel VIII^{#} umsetzt. 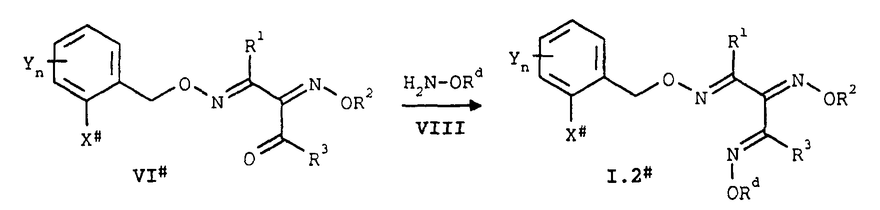
6. Die Umsetzung der Carbonylverbindung VI^{#} in den Trisoximether I.2^{#} erfolgt üblicherweise bei Temperaturen von 10°C bis 120°C, vorzugsweise 20°C bis 85°C, in einem inerten organischen Lösungsmittel gegebenenfalls in Gegenwart einer Base [vgl. EP-A 386 561].
   Geeignete Lösungsmittel sind Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Methanol und Ethanol. Es können auch Gemische der genannten Lösungsmittel verwendet werden.
   Als Basen kommen allgemein anorganische Verbindungen, wie Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, Alkalimetall- und Erdalkalimetallalkoholate, wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Triisopropylethylamin und N-Methylpiperidin, Pyridin, substituierte Pyridine wie Collidin, Lutidin und 4-Dimethylaminopyridin, sowie bicyclische Amine in Betracht. Besonders bevorzugt werden Natriumhydrogencarbonat, Pyridin und Triethylamin.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, VIII in einem Überschuß bezogen auf VI^{#} einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=Cund C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die -N=CR¹-C(CR³R⁴)=NOR² Doppelbindungen werden im allgemeinen hinsichtlich ihrer Wirksamkeit die E,E-Isomere der Verbindungen I bevorzugt (Konfiguration bezogen auf den Rest -CH₂O- im Verhältnis zur -C(CR³R⁴)=NOR²-Gruppe, bzw. bezogen auf den Rest -OR² im Verhältnis zur -C(R¹)-N=OCH₂-Gruppe).

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4 oder 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 Kohlenstoffatomen (wie vorstehend genannt), welche über ein Sauerstoffatom (-O-) an das Gerüst gebunden sind;
**Alkenyl:** ungesättigte, geradkettige oder verzweigte Kohlenwasserstoffreste mit 3 bis 4 Kohlenstoffatomen und einer Doppelbindung in einer beliebigen Position, z.B. C₃-C₄-Alkenyl wie 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl;
**Halogenalkenyl:** ungesättigte, geradkettige oder verzweigte Kohlenwasserstoffreste mit 3 bis 4 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 3 bis 4 Kohlenstoffatomen und einer Dreifachbindung in einer beliebigen Position, z.B. C₃-C₄-Alkinyl wie Ethinyl, 1-Propinyl, 2-Propinyl, 1-Butinyl, 2-Butinyl, 3-Butinyl und 1-Methyl-2-propinyl;
**Halogenalkinyl:** ungesättigte, geradkettige oder verzweigte Kohlenwasserstoffreste mit 3 bis 4 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;

Der Zusatz *"ggf. subst"* in Bezug auf den Phenylrest soll zum Ausdruck bringen, daß dieser Rest partiell oder vollständig halogeniert sein kann [d.h. die Wasserstoffatome dieses Restes können teilweise oder vollständig durch gleiche oder verschiedene Halogenatome wie vorstehend genannt (vorzugsweise Fluor, Chlor oder Brom, insbesondere Fluor oder Chlor) ersetzt sein] und/oder einen bis vier (insbesondere einen bis drei) der folgenden Reste tragen können:
Halogen, Cyano, Nitro, C₁-C₄-Alkyl, C₁-C₄-Halogenalkyl,
C₁-C₄-Alkoxy, C₁-C₄-Halogenalkoxy, C₁-C₄-Alkylamino,
Di-C₁-C₄-alkylamino und C₁-C₄-Alkylthio.

Im Hinblick auf ihre bestimmungsgemäße Verwendung sind Verbindungen der Formel I, in denen R^{a} C₁-C₂-Alkyl oder C₁-C₂-Alkoxy bedeutet, besonders bevorzugt.

Besonders bevorzugt sind Verbindungen der Formel IB.

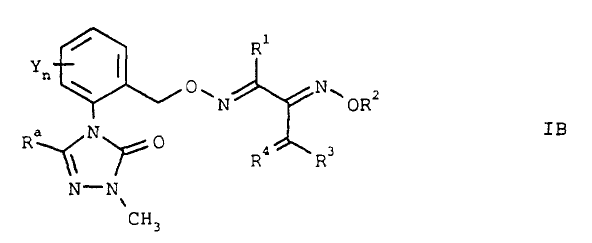

Ebenfalls bevorzugt sind Verbindungen der Formel I, in denen n = null (0) ist.

Insbesondere werden Verbindungen der Formel I bevorzugt, in denen R^{a} für Methyl oder Methoxy steht.

Daneben sind Verbindungen der Formel I.1 bevorzugt.

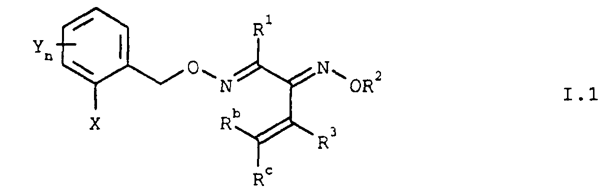

Gleichermaßen bevorzugt sind Verbindungen I.1. in denen R^{b} für _ Wasserstoff steht.

Weiterhin bevorzugt sind Verbindungen der Formel I.2.

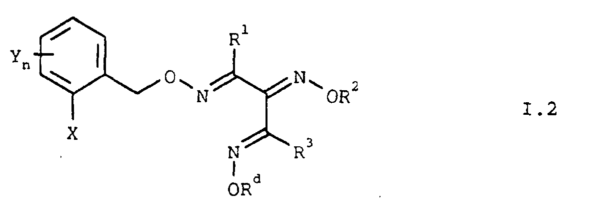

Insbesondere werden Verbindungen I.1 bevorzugt, in denen R³ und Yₙ für Wasserstoff und R² für Methyl oder Ethyl steht.

Außerdem werden Verbindungen I.1 besonders bevorzugt, in denen R³ für Wasserstoff, R² für Methyl oder Ethyl und Yₙ für 6-Methyl steht.

Gleichermaßen besonders bevorzugt sind Verbindungen I.2, in denen R¹ und R³ für Methyl, Yₙ für Wasserstoff und R² für Methyl oder Ethyl steht.

Daneben werden Verbindungen I.2 besonders bevorzugt, in denen Yₙ für 6-Methyl steht.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten*, *Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia inaequalis (Schorf) an Äpfeln.

Die Verbindungen I eignen sich außerdem zur Bekämpfung von Schadpilzen wie Paecilomyces variotii im Materialschutz (z.B. Holz, Papier, Dispersionen für den Anspruch, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren, sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergieroder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink- (N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid;
- N-Dichlorfluormethylthio-N',N' -dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan;
- Amine wie 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, (8-(1,1-Dimethylethyl)-N-ethyl-N-propyl-1,4-dioxaspiro[4.5]decan-2-methanamin;
- Azole wie 1-[2-(2,4-Dichlorphenyl)-4-ethyl-1,3-dioxolan-2-ylethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, 1-[2-(2,4-Dichlorphenyl)-pentyl]-1H-1,2,4-triazol, 2,4'-Difluor-a-(1H-1,2,4-triazolyl-1-methyl)-benzhydrylalkohol, 1-((bis-(4-Fluorphenyl)-methylsilyl)-methyl)-1H-1,2,4-triazol, 1-[2RS,4RS;-2RS,4SR)-4-brom-2-(2,4-dichlorphenyl)tetrahydrofuryl]-1H-1,2,4-triazol, 2--(4-Chlorphenyl)-3-cyclopropyl-1-(1H-1,2,4-triazol-1-yl)-butan-2-ol, (+)-4-Chlor-4-[4-methyl-2-(1H-1,2,4-triazol-1-ylmethyl)-1,3-dioxolan-2-yl]-phenyl-4-chlorphenylether, (E)-(R,S)-1-(2,4-dichlorphenyl)-4,4-dimethyl-2-(1H-1,2,4-triazol-1-yl)pent-1-en-3-ol, 4-(4-Chlorphenyl)-2-phenyl-2-(1H-1,2,4,-triazolylmethyl)-butyronitril, 3-(2,4-dichlorphenyl)-6-fluor-2-(1H-1,2,4-triazol-1-yl)chinazolin-4(3H)-on, (R,S)-2-(2,4-Dichlorphenyl)-1-H-1,2,4-triazol-1-yl)-hexano-2-ol, (1RS,5RS;1RS,5SR)-5-(4-chlorbenzyl)-2,2-dimethyl-1-(1H-1,2,4-triazol-1-ylmethyl)cyclopentanol, (R,S)-1-(4-chlorphenyl)-4,4-dimethyl-3-(1H-1,2,4-triazol-1-ylmethyl)pentan-3-ol, (+)-2-(2,4,-Dichlorphenyl)-3-(1H-1,2,4-triazolyl)-propyl-1,1,2,2-tetrafluorethylether, (E)-1-[1-[4-Chlor-2-trifluormethyl)-phenyl]-imino)-2-propoxyethyl]-1H-imidazol, 2-(4-Chlorphenyl)-2-(1H-1,2,4-triazol-1-ylmethyl)-hexannitril;
- α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol;
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid;
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin;
- Phenylpyrrole wie 4-(2,2-Difluor-1,3-benzodioxol-4-yl)-pyrrol-3-carbonitril;
- Zimtsäureamide wie 3-(4-Chlorphenyl)-3-(3,4-dimethoxyphenyl)-acrylsäuremorpholid;
- sowie verschiedene Fungizide, wie Dodecylguanidinacetat, 3-[3-(3,5-Dimethyl-2-oxycyclohexyl)-2-hydroxyethyl]-glutarimid, N-Methyl-, N-ethyl-(4-trifluormethyl, -2-[3',4'-dimethoxyphenyl]-benzoesäureamid, Hexachlorbenzol, DL-Methyl-N-(2,6-dimethyl-phenyl)-N-furoyl(2)-alaninat, DL-N-(2,6-Dimethyl-phenyl)-N- (2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl (-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, N-(3-Chlor-2,6-dinitro-4-trifluormethyl-phenyl)-5-trifluormethyl-3-chlor-2-aminopyridin.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: Herstellung von

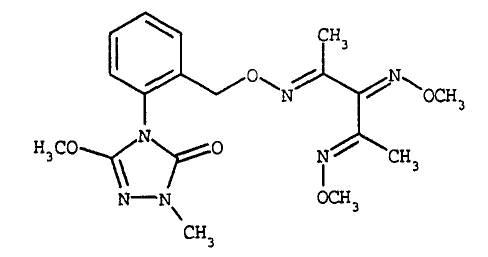

### Methode A:

Eine Lösung von 1,1 g 4-[(2-Brommethyl)phenyl]-2,4-dihydro-5-methoxy-2-methyl-3H-1,2,4-triazol-3-on [vgl. WO-A 97/00,612], 1,35 g Kaliumcarbonat und 0,68 g 2,3,4-Pentantrion-2-(E)-3-(Z)-bis(O-methyloxim)-4-(E)-Oxim [vgl. DE-A 195 39 324] in 35 ml Dimethylformamid (DMF) wurde 8 Std. bei 25-35°C gerührt. Dann wurde die Reaktionsmischung auf Eiswasser gegeben, mit Methyl-tert. butylether extrahiert und die vereinigten organischen Phasen nach Waschen mit ges. Ammoniumchlorid-Lösung getrocknet. Nach Abdestillieren des Lösungsmittels und Chromatographie des Rückstandes an Kieselgel (Cyclohexan/Essigester 80:20) erhielt man 400 mg Produkt als helles Pulver vom Schmp. 142-145°C.
IR (cm-1): 1715, 1611, 1502, 1481, 1415, 1049, 999, 988, 895.

### Methode B:

1,1 g 4-[(2-Brommethyl)phenyl]-2,4-dihydro-5-chlor-2-methyl-3H-1,2,4-triazol-3-on [vgl. WO-A 97/19935, Tabelle B, S. 23, Verb. Nr. 77], in 15 ml DMF gelöst, wurden mit 1,35 g Kaliumcarbonat und 0,68 g 2,3,4-Pentantrion-2-(E)-3-(Z)-bis(O-methyloxim)-4-(E)-Oxim [vgl. DE-A 195 39 324] etwa 12 Std. bei 22-25°C gerührt. Dann wurde die Reaktionsmischung auf Eiswasser gegeben und mit Methyl-tert. butylether extrahiert und die vereinigten organischen Phasen nach Waschen mit ges. Ammoniumchlorid-Lösung getrocknet. Nach Abdestillieren des Lösungsmittels wurde der Rückstand an Kieselgel (Cyclohexan/Essigester 80:20) chromatographiert. Das Rohprodukt (700 mg) wurde in 5 ml Dimethoxyethan und 5 ml Methanol gelöst und mit 0,61 g einer 30%igen methanolischen Natriummethylat-Lösung versetzt. Das Reaktionsgemisch wurde etwa 20 Std. unter Rückfluß erhitzt, in Methylenchlorid aufgenommen, dann mit Wasser und ges. Ammoniumchlorid-Lösung gewaschen, dann getrocknet. Nach Abdestillieren des Lösungsmittels und Chromatographie des Rückstandes an Kieselgel (Cyclohexan/Essigester 80:20) erhielt man 300 mg der Titelverbindung als helles Pulver vom Schmp. 142-145°C.
IR (cm-1): 1715, 1611, 1502, 1481, 1415, 1049, 999, 988, 895.

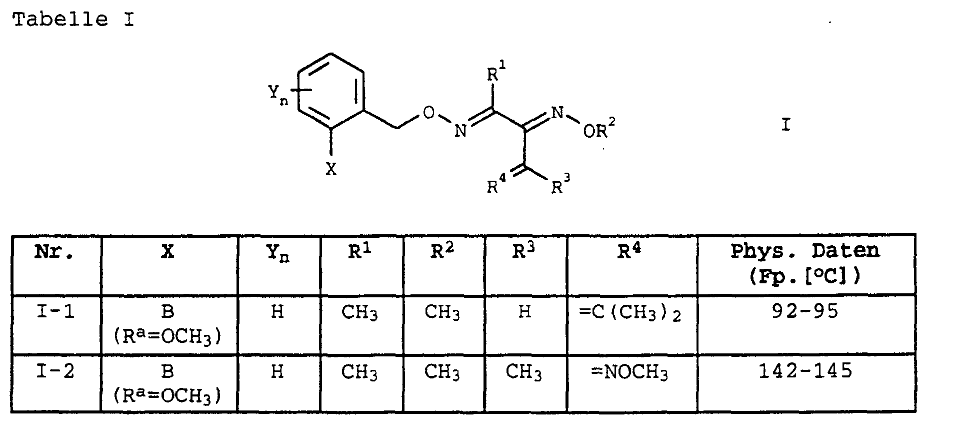

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen Weizenmehltau

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Erysiphe graminis forma specialis tritici) bestäubt. Die Versuchspflanzen wurden anschließend im Gewächshaus bei Temperaturen zwischen 20 und 24°C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

### Anwendungsbeispiel 2 - Wirksamkeit gegen Pyricularia oryzae (protektiv)

Blätter von in Töpfen gewachsenen Reiskeimlingen der Sorte "Tai-Nong 67" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Am folgenden Tag wurden die Pflanzen mit einer wäßrigen Sporensuspension von Pyricularia oryzae inokuliert. Anschließend wurden die Versuchspflanzen in Klimakammern bei 22 - 24°C und 95 - 99 % relativer Luftfeuchtigkeit für 6 Tage aufgestellt. Dann wurde das Ausmaß der Befallsentwicklung auf den Blättern visuell ermittelt.

In diesen Tests zeigten die mit 16 ppm der Verbindung Nr. I-1 der Tabelle I behandelten Pflanzen maximal 5 % Befall, während die Unbehandelten zu 80, bzw. 85 % befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

### Die Wirkstoffe wurden

a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
   aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Bisiminosubstituierte Phenylverbindungen der Formel I, 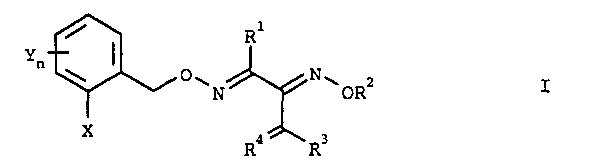 in der die Substituenten folgende Bedeutung haben:
x eine Gruppe A oder B, 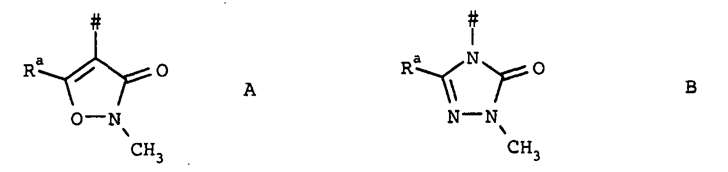 wobei
# die Bindung mit dem Phenylring kennzeichnet und
R^{a} für Halogen, C₁-C₄-Alkyl oder C₁-C₄-Alkoxy steht;
Y Halogen, C₁-C₄-Alkyl, C₁-C₄-Halogenalkyl oder C₁-C₄-Alkoxy;
n 0, 1 oder 2, wobei die Reste Y verschieden sein können, wenn n = 2 ist;
R¹ C₁-C₄-Alkyl;
R² C₁-C₄-Alkyl, C₁-C₄-Halogenalkyl, C₃-C₄-Alkenyl, C₃-C₄-Halogenalkenyl, C₃-C₄-Alkinyl oder C₃-C₄-Halogenalkinyl;
R³ Wasserstoff, C₁-C₄-Alkyl, C₁-C₄-Halogenalkyl, Phenyl oder partiell oder vollständig halogeniertes Phenyl oder Phenyl mit 1-4 der folgenden Reste: Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio;
R⁴ =CR^{b}R^{c} oder =N-OR^{d}, wobei
R^{b}, R^{c} unabhängig voneinander Wasserstoff, C₁-C₆-Alkyl, Phenyl oder partiell oder vollständig halogeniertes Phenyl oder Phenyl mit 1-4 der folgenden Reste: Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio bedeuten; und
R^{d} einer der bei R² genannten Reste ist.

2. Verfahren zur Herstellung der Verbindungen der Formel I.1 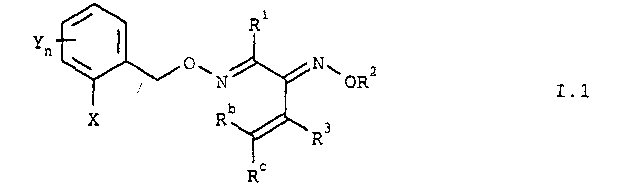 nach Anspruch 1, **dadurch gekennzeichnet, daß** man eine Benzylverbindung der Formel II, 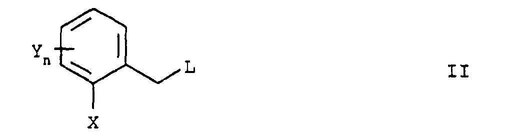 in der L für eine nucleophil austauschbare Gruppe steht, mit einem Oxim der Formel III 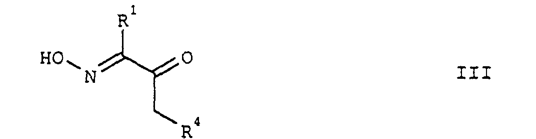 umsetzt und den erhaltenen Oximether der Formel IV 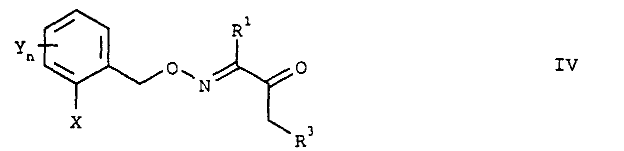 durch Halogenierung in die Halogenverbindung der Formel V 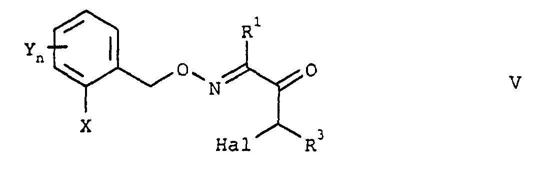 überführt, V mit einem Hydroxylaminether der Formel VI'
H₂N―OR² VI'
zum Bisoxim der Formel VI, 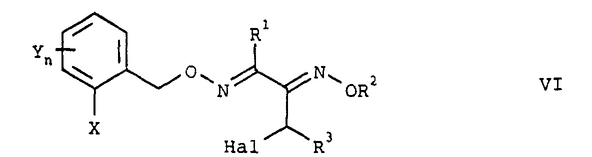 umsetzt, VI zur Carbonylverbindung der Formel VII 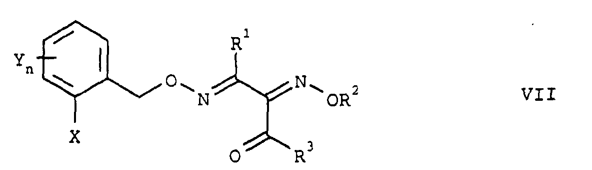 oxidiert und VII mit einem Phosphor-Reagenz im Sinne einer Wittig-Reaktion zu einer Verbindung der Formel 1.1 umsetzt.

3. Verfahren zur Herstellung einer Verbindung der Formel I.2 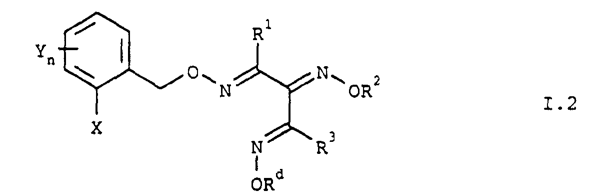 nach Anspruch 1, **dadurch gekennzeichnet, daß** man eine Carbonylverbindung der Formel VI nach Anspruch 2 mit einem Hydroxylaminether der Formel VIII
H₂N-OR^{d} VIII
umsetzt.

4. Zwischenprodukte der Formel VII gemäß Anspruch 2

5. Zur Bekämpfung von tierischen Schädlingen oder Schadpilzen geeignetes Mittel, enthaltend einen festen oder flüssigen Trägerstoff und eine Verbindung der Formel I gemäß Anspruch 1.

6. Verwendung der Verbindungen I gemäß Anspruch 1 zur Herstellung eines zur Bekämpfung von tierischen Schädlingen oder Schadpilzen geeigneten Mittels.

7. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

8. Verfahren zur Bekämpfung von tierischen Schädlingen, **dadurch gekennzeichnet, daß** man die tierischen Schädlinge oder die vor ihnen zu schützenden Materialien, Pflanzen, den Boden oder Saatgüter mit einer wirksamen Menge einer Verbindung der Formel I gemäß Anspruch 1 behandelt.

## Claims

1. A bisimino-substituted phenyl compound of the formula I 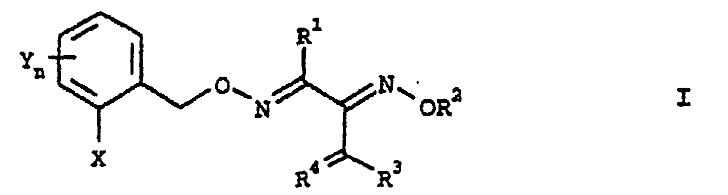 where the substituents have the following meanings:
x is a group A or B 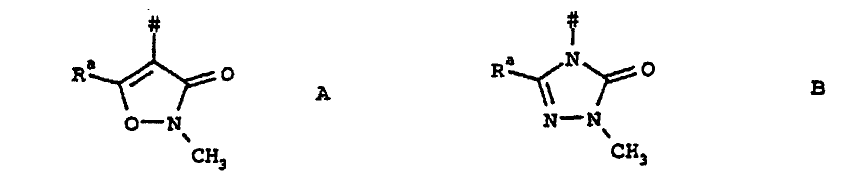 where
# denotes the bond with the phenyl ring and
R^{a} is halogen, C₁-C₄-alkyl or C₁-C₄-alkoxy;
Y is halogen, C₁-C₄-alkyl, C₁-C₄-haloalkyl or C₁-C₄-alkoxy;
n is 0, 1 or 2, it being possible for the radicals Y to be different if n = 2;
R¹ is C₁-C₄-alkyl;
R² is C₁-C₄-alkyl, C₁-C₄-haloalkyl, C₃-C₄-alkenyl, C₃-C₄-haloalkenyl, C₃-C₄-alkynyl or C₃-C₄-haloalkynyl;
R³ is hydrogen, C₁-C₄-alkyl, C₁-C₄-haloalkyl phenyl or partially or completely halogenated phenyl or phenyl having 1-4 of the following radicals cyano, nitro, C₁-C₄-alkyl, C₁-C₄ haloalkyl, C₁-C₄-alkoxy, C₁-C₄-haloalkoxy, C₁-C₄-alkylamino, di- C₁-C₄-alkylamino and C₁-C₄-alkylthio;
R⁴ is =CR^{b}R^{c} or =N-OR^{d} where
R^{b}, R^{c} independently of one another are hydrogen, C₁-C₆-alkyl phenyl or partially or completely halogenated phenyl or phenyl having 1-4 of the following radicals cyano, nitro, C₁-C₄-alkyl, C₁-C₄ haloalkyl, C₁-C₄-alkoxy, C₁-C₄-haloalkoxy, C₁-C₄-alkylamino, di- C₁-C₄-alkylamino and C₁-C₄-alkylthio; and
R^{d} is one of the radicals mentioned under R².

2. A process for the preparation of a compound of the formula I.1 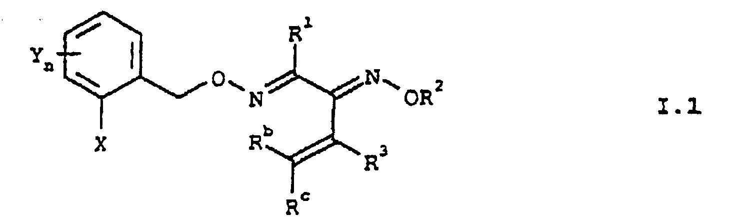 as claimed in claim 1, which comprises reacting a benzyl compound of the formula II 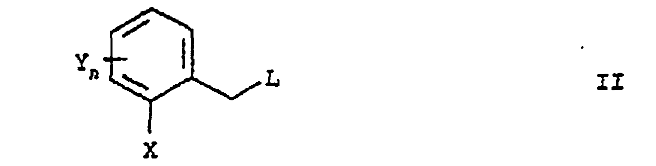 where L is a nucleophilically exchangeable group with an oxime of the formula III 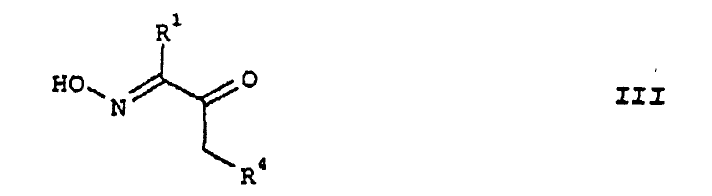 and converting the resulting oxime ether of the formula IV 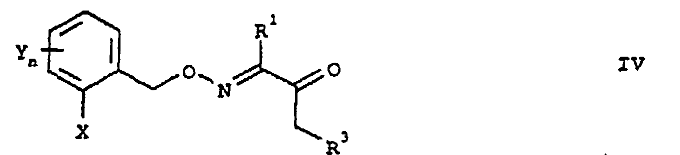 into the halogen compound of the formula V 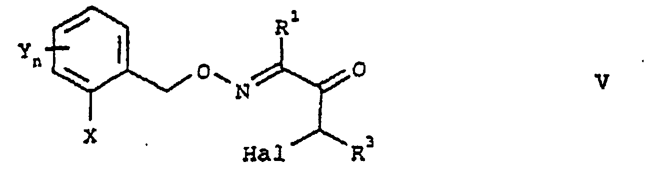 by means of halogenation, reacting V with a hydroxylamine ether of the formula VI'
H₂N-OR² VI'
to give the bisoxime of the formula VI, 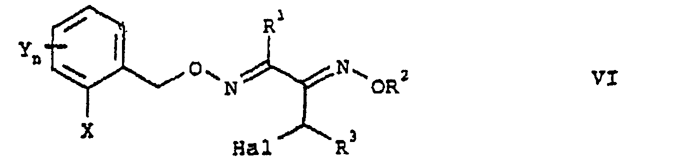 oxidizing VI to give the carbonyl compound of the formula VII 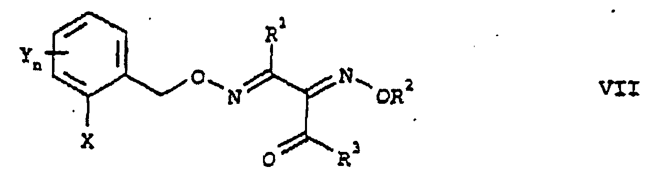 and reacting VII with a phosphorus reagent following the principles of a Wittig reaction to give a compound of the formula I.1.

3. A process for the preparation of a compound of the formula I.2 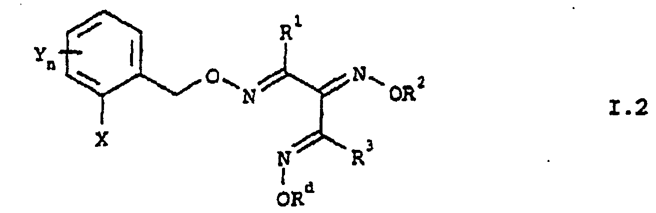 as claimed in claim 1, which comprises reacting a carbonyl compound of the formula VI as set forth in claim 2 with a hydroxylamine ether of the formula VIII
H₂N-OR^{d}

4. An intermediate of the formula VII as set forth in claim 2.

5. A composition which is suitable for controlling animal pests or harmful fungi, comprising a solid or liquid carrier and a compound of the formula I as claimed in claim 1.

6. The use of the compounds I as claimed in claim 1 for the preparation of a composition which is suitable for controlling animal pests or harmful fungi.

7. A method of controlling harmful fungi, which comprises treating the fungi, or the materials, plants, the soil or seeds to be protected against fungal infection, with an effective amount of a compound of the formula I as claimed in claim 1.

8. A method of controlling animal pests, which comprises treating the animal pests, or the materials, plants, the soil or seeds to be protected against them, with an effective amount of a compound of the formula I as claimed in claim 1.

## Revendications

1. Composés phényliques bisimino-substitués de formule I, 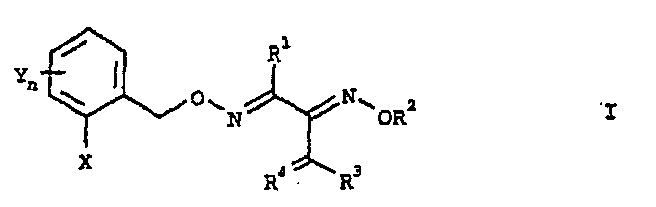 dans laquelle les substituants ont la signification suivante :
x représente un groupe A ou B, 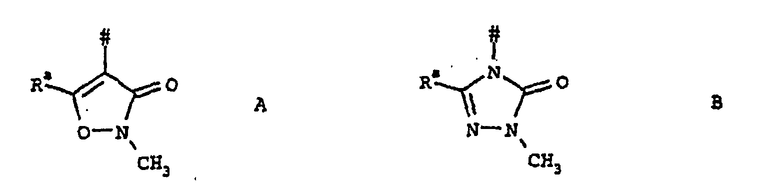 dans lequel
# caractérise la liaison au noyau phénylique et
R^{a} représente un halogène, un alkyle en C₁-C₄ ou un alcoxy en C₁-C₄ ;
Y représente un halogène, un alkyle en C₁-C₄, un halogénoalkyle en C₁-C₄ ou un alcoxy en C₁-C₄ ;
n vaut 0, 1 ou 2, les radicaux Y pouvant être différents, si n = 2 ;
R¹ représente un alkyle en C₁-C₄ ;
R² représente un alkyle en C₁-C₄, un halogénoalkyle en C₁-C₄, un alcényle en C₃-C₄, un halogénoalcényle en C₃-C₄, un alcynyle en C₃-C₄ ou un halogénoalcynyle en C₃-C₄ ;
R³ représente un hydrogène, un alkyle en C₁-C₄, un halogénoalkyle en C₁-C₄, un phényle ou un phényle partiellement ou totalement halogéné ou un phényle ayant 1-4 des radicaux suivants : un cyano, un nitro, un alkyle en C₁-C₄, un halogénoalkyle en C₁-C₄, un alcoxy en C₁-C₄, un halogénoalcoxy en C₁-C₄, un alkylamino en C₁-C₄, un di-C₁-C₄-alkylamino et un alkylthio en C₁-C₄ ;
R⁴ représente =CR^{b}R^{c} ou =N-OR^{d}, où
R^{b}, R^{c} représentent, indépendamment l'un de l'autre, un hydrogène, un alkyle en C₁-C₆, un phényle ou un phényle partiellement ou totalement halogéné ou un phényle ayant 1-4 des radicaux suivants : un cyano, un nitro, un alkyle en C₁-C₄, un halogénoalkyle en C₁-C₄, un alcoxy en C₁-C₄, un halogénoalcoxy en C₁-C₄, un alkylamino en C₁-C₄, un di-C₁-C₄-alkylamino et un alkylthio en C₁-C₄ ; et
R^{d} est l'un des radicaux mentionnés pour R².

2. Procédé de préparation des composés de formule I.1 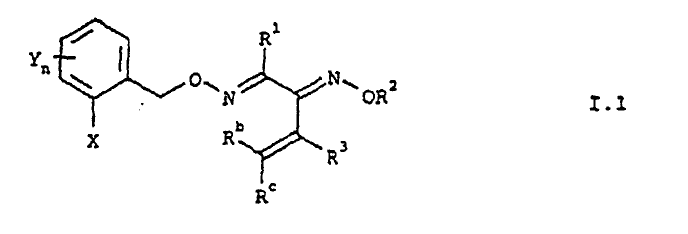 selon la revendication 1, **caractérisé en ce que** l'on fait réagir un composé benzylique de formule II, 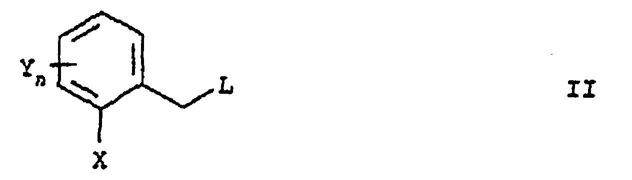 dans laquelle L représente un groupe échangeable par voie nucléophile, avec un oxime de formule III 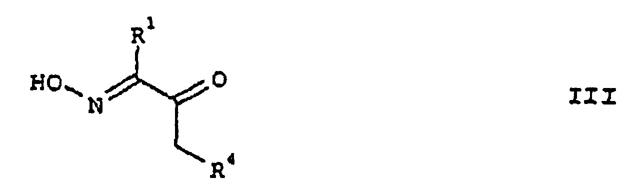 et on transforme l'oxime-éther obtenu de formule IV 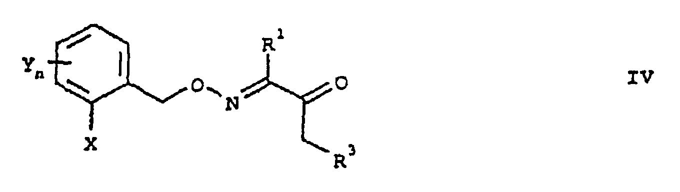 par halogénation en le composé halogéné de formule V, 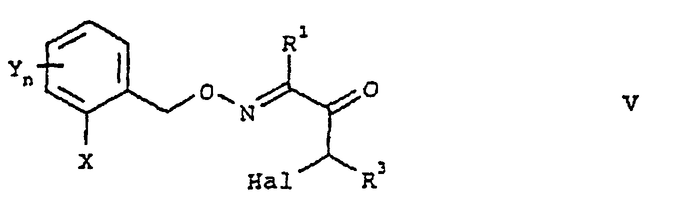 on fait réagir V avec un éther d'hydroxylamine de formule VI'
H₂N―OR² VI'
pour donner lieu au bisoxime de formule VI, 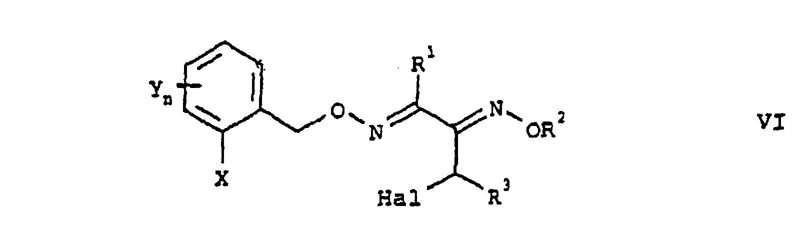 on oxyde VI pour donner lieu au composé carbonylé de formule VII 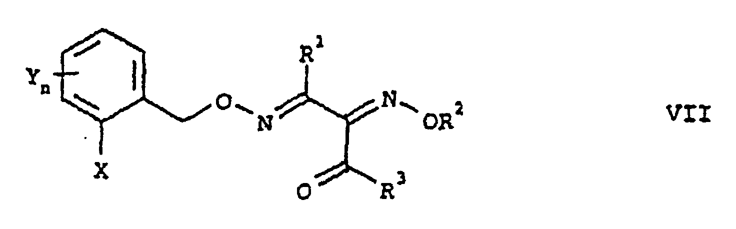 et on fait réagir VII avec un réactif phosphoré selon les principes d'une réaction de Wittig pour donner lieu à un composé de formule I.1.

3. Procédé de préparation d'un composé de formule I.2 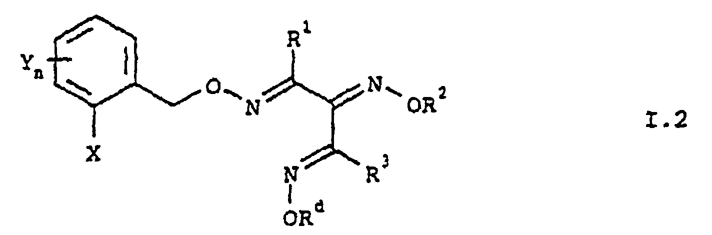 selon la revendication 1, **caractérisé en ce que** l'on fait réagir un composé carbonylé de formule VI selon la revendication 2 avec un éther d'hydroxylamine de formule VIII
H₂N―OR^{d} VIII.

4. Intermédiaires de formule VII selon la revendication 2.

5. Composition appropriée pour la lutte contre des parasites animaux ou des champignons nuisibles, comprenant un support solide ou liquide et un composé de formule I selon la revendication 1.

6. Utilisation des composés I selon la revendication 1 pour la préparation d'une composition appropriée pour la lutte contre des parasites animaux ou des champignons nuisibles.

7. Procédé de lutte contre des champignons nuisibles, **caractérisé en ce que** les champignons ou les matériaux, les plantes, les sols ou les semences à protéger vis-à-vis d'une infection fongique sont traités par une quantité efficace d'un composé de formule I selon la revendication 1.

8. Procédé de lutte contre des parasites animaux, **caractérisé en ce que** les parasites animaux ou les matériaux, les plantes, les sols ou les semences à protéger vis-à-vis de ceux-ci sont traités par une quantité efficace d'un composé de formule I selon la revendication 1.
